# EUROPEAN PATENT APPLICATION

(11) **EP 2 550 986 A1**
(43) Date of publication of application: **30.01.2013**
(21) Application number: 11175155.8
(22) Date of filing: 25.07.2011
(51) Int. Cl.: A61M 15/08

(54) **Device for applying chemical substances**

(71) Applicant: A & K Film und Touristik GmbH, 14558 Nuthetal (DE)
(72) Inventor: Fonfé, Hans, 14558 Nuthetal (DE)

(57) **Abstract**

The invention refers to a device (10) for applying a volatile chemical substance into a body opening of a subject. According to the invention, the device (10) comprises a hollow body (1), with a first opening (2a) and a second opening (2b) at each end and an adjustment means for adjusting to different lengths of nasal canals and preventing the device (10) from falling out of the nose.

## Description

The present invention relates to a device for applying a volatile chemical substance such as a drug into a body opening, preferably into the nose of a subject, in particular a human subject.

### Introduction

Diseases of the nasopharyngeal zone are often accessible for treatment through the nose. The application of chemical substances, such as drugs, for treating diseases such as common colds, allergies, respiratory infections or snoring and/or alleviating the symptoms of these diseases is often performed using liquids and ointments.

However, liquids have the tendency to run out the body opening when the subject assumes a position that allows the liquid to follow gravitational pull. The application of ointments can be disadvantageous if the ointment containing the drug comes in contact with the mucous membrane of the subject, leading to irritation, bleeding or allergic reactions.

Therefore, there is a need in the art to allow for the application of a chemical substance into a body opening that avoids at least in part the disadvantageous described above.

### Description of the invention

The device of the invention comprises a hollow body and an adjustment means. The hollow body is configured to contain a volatile chemical substance and can at least in part be inserted into a body opening of a subject, preferably the nose.

The hollow body of the device has a first opening at a first end of the hollow body, and a second opening at a second end of the hollow body. The adjustment means is for adjusting the position of the device in the body opening, for ensuring fitting of the device in the body opening and for adjusting penetration depth of the device in the body opening.

The hollow body has at least an inner wall and an outer wall. The adjustment means are positioned at the outer wall of the hollow body of the device. If the hollow body is e.g. in the form of a cylinder, the outer wall is the outer circumference of the hollow body. The adjustment means allows fitting the device to the size and shape of the body opening of the subject using the device and prevents the device from falling out of the body opening, e.g. out of the nose. The adjustment means also ensures that the device is not inserted into the body opening too far. Instead, it ensures that part of the device will remain outside the body of the subject, allowing easy removal of the device.

The device can be inserted into the body opening, e.g. nose of a subject. The subject is a mammal, preferably an animal or a human, male or female. The device can be used by children and adults. It can also be used to apply a chemical substance to an animal in need of such application.

The outer wall of the hollow body of the device is preferably smooth. This ensures that upon insertion into the body opening, the mucus membrane of the subject are not irritated or perforated during use.

In a preferred embodiment of the device, the first opening and the second opening are arranged such as to allow an air passage through the device. This allows for a volatile chemical substance that is located within the hollow body of the device, as will be explained below, to evaporate and to be moved into the body opening of the subject. When the device is to be used for the nose, airflow through the device allows the subject to breathe. Also, the volatile chemical substance can enter the body opening of the subject, in particular the nose with the airflow.

The device can be produced in various shapes and sizes, as the main functionality of the device is to allow vapors into body openings, such as the nasal passage. Using it for inserting it into the nose, the preferred shape of the hollow body is a cylindrical or conical shape.

In general, a cylinder, a possible form of the hollow body, is defined more broadly as any ruled surface spanned by a one-parameter family of parallel lines. Cross sections of a cylinder can be, for example, ellipse, hyperbola or parabola.

It is preferred that the hollow body of the device has the shape of a hollow cylinder or of a hollow cone, preferably of a hollow truncated cone to avoid injury upon insertion into the body opening. In a preferred embodiment, the hollow body of the device is a right circular cylinder, in particular if the device is to be used for insertion into the nose.

The shape of the device of the invention, as well as the vapor escaping from a volatile chemical substance such as an essential oil, open up the airways when inserted into the nose, helping the subject to breathe more easily. It was also found by the inventor, that the use of the device also improves or even eradicates snoring. Therefore, when inserted into the nose of a subject, the device serves as an anti-snoring aid. If necessary, the device can remain inside the nose during nighttime or when needed.

Openings alongside the circumference of the hollow body (i.e. the part that contacts the skin or mucous membrane of the subject) are preferably not present to prevent the chemical substance or the carrier substance carrying the essential oils to contact the mucus membrane of the subject, as this may cause irritation and adverse effects. Instead, the hollow body is made in one piece with a continuous wall.

In another preferred embodiment, the hollow body is frusto-conical in shape. In this case, the movable ring has to be stretchable, to be able to be moved along the longitudinal axis of the frusto-conical surface of the hollow body, while touching the surface. If the increase in diameter is relatively small along a longitudinal axis of the frusto-conical hollow body of the device, silicone can be used, since silicone elastomers are stretchable. If the ratio of diameter from first to second opening of the hollow body decreases rather sharply, elasticity of the silicone ring has to be increased accordingly.

As a further measure to prevent injury, the hollow body tapers down at the first end and/or at the second end of the hollow body to form a rounded tip. The size of the holes at the first and second end of the hollow body should be wide enough to allow a sufficient airflow for breathing and/or allowing the vapor from the volatile chemical substance to move into the body opening. It should be mentioned that the subject using the device usually will be able to breathe through the nostril that does not contain the device, even if the holes are chosen to be rather small.

It is preferred that the adjustment means is a movable ring. The movable ring can be positioned on the outer wall of the hollow body such that it is movable on the outer wall of the hollow body, preferably along a longitudinal axis of the hollow body. Sliding the ring along the longitudinal extension of the hollow body allows for the adjustment of the penetration depth into the body opening of the subject using the device. Sliding the movable ring is particularly easy if both the movable ring and the hollow body it embraces are made of medical silicone.

In a preferred embodiment of the device, the movable ring comprises a tongue for engaging into a groove that is positioned along a longitudinal axis of the hollow body. Accordingly, the tongue of the adjustment means can slide along the groove, such that the movable ring can be adjusted in any position along the groove. It is preferred that the groove is positioned on the hollow body starting at a first area close to the first end of the hollow body, stretching to a second area close to the second end of the hollow body. The groove may be a depression in the wall of the hollow body or may be a recess that extends into the hollow inner part of the hollow body.

The position of groove and tongue are interchangeable, i.e. the tongue can also be positioned longitudinally along the side of the surface of the hollow body and a groove can be positioned at the inner side (i.e. facing the outer wall of the hollow body) of the movable ring, such that the tongue engages into the groove and allows the movable ring to slide along a longitudinal axis of the hollow body of the device.

In an alternative preferred embodiment, the adjustment means is an adhesion means such as an adhesive point and/or strip on the outer wall of the hollow body. This adhesion means also serves for affixing the device in the body opening of the subject. An adhesion means in the form of e.g. a glue point can be located in an area near the first or second end of the hollow body such that it can be attached to an area of skin outside the body opening, so that the adhesive does not come into contact with the mucous membrane of the subject. When the device is inserted into the nose, the adhesion means is located such on the hollow body of the device that it contacts the skin blow the nose of the subject with the part of the device that is not inserted into the nose.

In order for the device to be filled and contain a chemical substance, such as a medicine, an absorbing material may be located within the hollow body of the device. This absorbing material can absorb a chemical substance, in particular in form of a liquid. The absorbing material can be an absorbing porous filter, cotton, felt, or a similar material.

The absorbing material can be removed after use, and fresh absorbing material can then be inserted into the hollow body. This insertion is preferably performed through the first and/or the second opening in the hollow body. A volatile chemical substance in form of a liquid can then be applied onto the absorbing material prior to use of the device.

Alternatively, the device of the invention may comprise a carrier substance within the hollow body. The carrier substance binds liquid chemical substances or liquids containing chemical substances that are to be administered to the subject. Petroleum jelly (Vaseline^{®}), udder ointment, wax, or lanolin can be used as carrier substances. Preferred liquids are essential oils that can be mixed into the carrier substance prior to use of the device. This way, the chemical substance in the form of a liquid can be inserted into the hollow body of the device without flowing out of the device. Essential oils are volatile.

Optionally, a removable cap is located at the first and/or at the second end of the hollow body. The presence of a cap at one or at both ends of the hollow body prevents the volatile substance to escape from the device when not in use. This allows storage of the device when a volatile chemical substance is present in the device.

The device can comprise or consist of an organic or an inorganic material that will not cause adverse reactions of the subject's body and is soft enough to prevent injury of the subject upon insertion. Preferably, the device comprises or consists of medical silicone.

Adhesive means can be made off skin-friendly material, strong enough to keep the device in place during the course of application, e.g. over night, but also easy to remove when the device is not in use any longer.

If the device consists of medical silicone, an additional adhesive strip can preferably be affixed to the surface of the device such that the strip wraps around the entire circumference of the outer surface of the hollow body. The width of the adhesive strip may be chosen according to the size of a glue point that is to be place on the adhesive strip. Preferably, a double-sided adhesive strip in the shape of a circle, providing an area the same size or bigger than the diameter of the glue points, is attached to or around the hollow body's outer wall.

The device combines the mechanical attribute of opening up the nasal passages with medical benefits of essential oils, having a positive impact on possibly firming the soft palate tissue.

The main hollow body of the device can be filled with essential oils in different ways. E.g., a filter, consisting of porous material, may be inserted into the hollow body. A required amount of drops of a liquid containing a volatile chemical substance for treating a discomfort or a disease, such as an essential oil, is being dripped onto the filter.

Essential oils or a blend of essential oils that might be used are obtained from thyme, mint, eucalyptus, rosemary, lavender, chamomile and/or onion.

Alternatively, filling may be achieved with a carrier substance and at least one essential oil mixed together prior to insertion. The mixture may be inserted into the hollow body of the device, e.g. with a spatula-like device, a small plastic spoon, or with a syringe. By squeezing the device from the outside, the carrier substance may be removed from the device.

The carrier substance, such as petroleum jelly, udder ointment, wax, or lanolin, is preferably non-toxic, skin friendly and well tolerated by the nasal mucous membranes or by the ear mucous membrane. Good results concerning mixing and keeping essential oils contained and long lasting, petroleum jelly is preferably used for mixing with essential oils.

Essential oils might help firm tissues of soft palate. After consistent use, the effect of firming the tissue, can lead to less snoring.

For storage purposes and to prevent vapor from escaping the hollow body, a movable cap is placed over the first and/or second end of the hollow body of the device. The movable cap can be made of the same material as the hollow body, such as an organic or an inorganic material that will not cause adverse reactions of the body, preferably silicone.

The production of medical silicone pieces follow standard processes that are known in the art, which may be injection molding of liquid silicone rubber, followed by vulcanization. The device can be made in any desired sizes, depending on the subject and the purpose of use.

Specifically, the process for producing a device described herein comprising molding of a hollow body for containing a volatile chemical substance for inserting into a body opening of a subject, with a first opening at a first end of the hollow body, and a second opening at a second end of the hollow body, and an adjustment means for adjusting the position of the device in the body opening.

The invention also refers to the use of a device described herein for applying a volatile chemical substance into a body opening of a subject.

### Examples

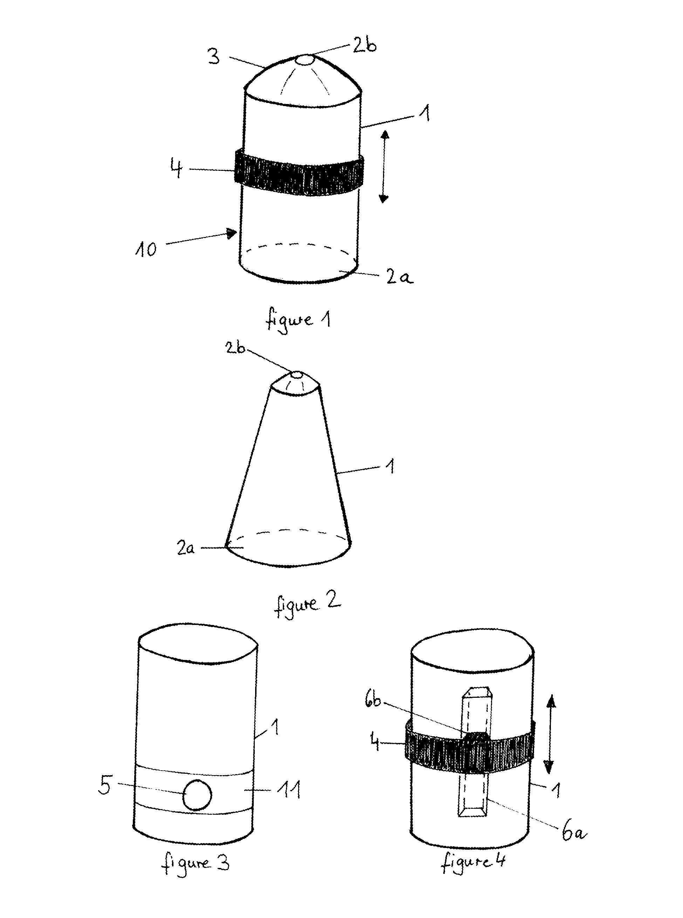

A series of tests with 100 subjects were performed and evaluated with a device as shown in figure 1 containing a porous filter holding a mixture of essential oils for preventing snoring. The mixture of essential oils could be re-applied when required.

The test was administered over a time span of 7 days as an anti-snoring aid at night. All subjects were tested for the following criteria: comfort of wear, adjustability, slipping and medical use.

The subjects were picked to represent two different age groups, female and male, favoring the older and male subjects. The distribution is as follows: 40 subjects, male, ages 40 - 70 years; 30 subjects, male, 25 - 39 years; 20 subjects, female, 40 - 75 years; 10 subjects, female, 25 - 39 years

Test results showed 70 % of all subjects were satisfied with wear and medical usage of the device, reporting they stopped snoring from the first test day on while using the device. 17 % of all subjects showed poor compliance and did not participate in the test until the end. 10 % of all subjects were not satisfied with the wear of the device. 3 % of all subjects abandoned the test due to high concentration of essential oils used. Overall, 60 % of all subjects tested would buy the device and use it on a regular basis.

### Figures

Embodiments of the present invention will now be described, given as a non-restrictive example, with reference to the drawings, wherein:
Figure 1 is a perspective view of an embodiment of the device;
Figure 2 is a perspective view of an alternate embodiment of the invention;
Figure 3 shows a perspective view of an alternate embodiment with alternate adjusting and adjustment means;
Figure 4 shows a perspective view of another alternate embodiment with alternate adjusting and adjustment means;
Figure 5 shows a perspective view of the embodiment with filter;
Figure 6 shows a perspective view of the embodiment with a carrier substance and essential oils; and
Figure 7 is a perspective view of the embodiment with a removable cap.

With reference to the drawings, figures 1 - 7, a device 10 for applying a chemical substance into a body opening, in particular the nose or ear of a subject, is being described.

The device 10 can also be inserted into the subject's ear for treating ear infections and earaches. Inserting the device 10 into the ear with essential oils, such as from onions, can sooth pain and reduce inflammation. The device can therefore be used for applying a chemical substance to treat an ear disease.

The device 10 comprises of a hollow body 1, shown in figure 1, with a first opening 2a and a second opening 2b, located at the first and second end of the hollow body 1, respectively. Preferably, the hollow body 1 is of cylindrical shape, wherein both ends maintain the same diameter along a longitudinal axis of the hollow body 1.

In the example shown, the first opening 2a is of the same diameter as the hollow body 1, allowing porous material and a carrier substance 8, for absorbing liquids to be pushed through the first opening 2a.

In this invention, various ways of including filter 7 and carrier substance 8 are possible. The porous material, for example a filter 7, and a carrier substance 8, for example petroleum jelly, including a liquid volatile chemical substance, for example essential oils, may be inserted during production process. The porous material may also be inserted during the production process without the liquid material.

The second opening 2b at the end of the hollow body 1 is preferably smaller than the first opening 2a. This allows vapor of the essential oils to escape in a controlled, preferred direction into the subject's nose or ear.

Other openings are not provided to prevent essential oils from touching the sensitive areas of nose or ear.

To prevent injuries to the nasal mucous membranes or the ear canal the end of the hollow body 1 where the second opening 2b is located, closes down to a rounded tip 3 with the relatively small second opening 2b at its very tip.

The preferred material used is flexible, medical silicone.

Sizes of the hollow body 1 preferably range from 2 to 4 centimeters (cm) in length, 0.5 to 1.5 cm in outer diameter and 0.3 to 0.8 cm in inner diameter. In one embodiment, the length of the hollow body 1 is 3 cm, the outer diameter is 0.9 cm and the inner diameter 0.55 cm.

The adjustment means in form of a movable ring 4 is positioned on the hollow body 1. The ring 4 is snug fit on the hollow body 1, but free to move along the longitudinal axis of the hollow body 1, from one end to the other end of the hollow body 1. This feature allows the user to adjust for the correct placement of the device 10 in the nose or ear and also keeps the device 10 in place for a longer period of time, e.g. over night.

Another preferred embodiment, shown in figure 2, is a hollow body 1 in the shape of a truncated cone, wherein the diameter of the end with the first opening 2a is bigger than the other end with the second opening 2b. The gradient follows a longitudinal axis of the hollow body 1. A movable ring 4 with increased elasticity can be fitted over the hollow body 1 and is free to move along the longitudinal axis.

In another embodiment, shown in figure 3, an adhesive point 5 as adhesive means is attached to the surface of the hollow body 1, evenly distributed on the surface of the hollow body 1 to allow proper location and fit of the device 10. An adhesive point 5 is a preferred adjustment means for hollow bodies 1 of the shape of truncated cones or pear-shaped. The adhesive means are preferably located at the first or second end of the hollow body 1 such that they can be brought in contact with the skin of the subject using the device 10 outside of the body opening. When used in the nose, the adhesive means are preferably brought in contact with the skin below the nose. This way, irritations of the mucous membrane and nose bleeding are avoided.

If the device 10 consists of medical silicone, an additional adhesive strip 11 as an adhesive means can preferably be affixed to the outer surface of the device 10 such that the adhesive strip 11 wraps around the entire circumference of the outer wall's surface of the hollow body 1, due to the fact that the adhesive bonding of the medical silicone with the glue point 5 is not sufficient. The width of the adhesive strip 11 may be chosen according to the size of a glue point 5 that is to be place on the adhesive strip 11. Preferably, a double-sided adhesive strip 11 in the shape of a circle, providing an area the same size or bigger than the diameter of the glue point 5, is attached to the hollow body's 1 outer wall.

In a further embodiment, shown in figure 4, the hollow body 1 is constructed with a groove 6a along the longitudinal axis of the hollow body 1, preferably a cylinder. Integrated into the movable ring 3 is a tongue 6b allowing the ring 4 to move along the groove 6a. In another embodiment, not shown in this drawing, is the conversely arranged tongue on the cylinder and groove on the ring.

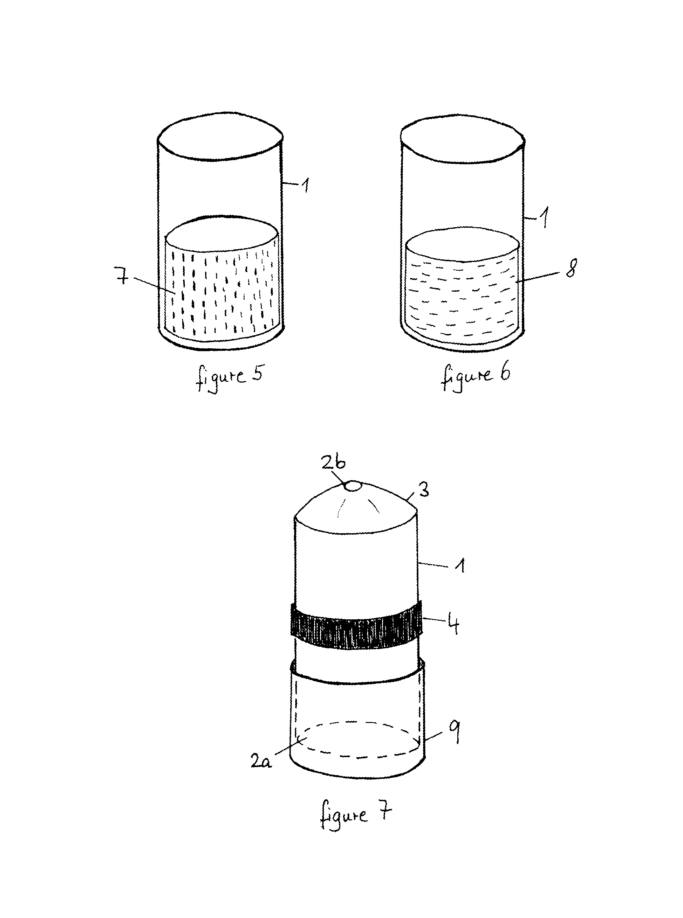

In order to absorb a liquid volatile chemical substance, such as essential oils, a filter 7 is positioned into the hollow body 1 in one embodiment, as shown in figure 5. The filter 7 comprises or consists of porous material, for example wood, foam, paper, fleece, and/or cellulose acetate. The cylindrical filter 1 is inserted through the first opening 2a and is almost of the diameter of the inner diameter of the hollow body 1. The length of the filter 7 shown here is about half the length of the hollow body 1 and ends preferably even with the first opening 2a.

Executing another method of binding liquid essential oils the hollow body 1 of the device 10, the hollow body 1, for example in the shape of a cylinder, is filled with a carrier substance 8 containing a liquid essential oil, wherein the substance may be applied through the first opening 2a. The carrier substance 8, containing the liquid essential oils, is located at the end of the first opening 2a, protruding preferably halfway into the hollow body 1. The carrier substance 8 consists preferably of petroleum jelly, udder ointment, wax and/or lanolin. Insertion of the carrier substance mixed with the essential oil can be introduced into the inside of the hollow body 1 using a spatula-like device, a small plastic spoon, or with a syringe.

In another embodiment, a combination of the filter 7 and carrier substance 8 located in the hollow body 1 at the same time, is included.

In order to seal the first opening 2a for storage purposes and to prevent vapor to escape, a removable cap 9 may also be supplied. The cap 9 can be slid over the first opening 2a.

The device can be used to treat or alleviate diseases such as common colds, allergies, respiratory infections and/or for treating or improving snoring. The use for preventing snoring is preferred.

### Reference signs

- 1: hollow body
- 2a: first opening
- 2b: second opening
- 3: rounded tip
- 4: adjustment means, movable ring
- 5: adjustment means, adhesive points
- 6a: groove
- 6b: tongue
- 7: porous material
- 8: carrier substance
- 9: removable cap
- 10: device
- 11: adhesive strip

## Claims

1. Device (10) for applying a volatile chemical substance into a body opening of a subject, comprising
- a hollow body (1) for containing a volatile chemical substance (for inserting into a body opening of a subject), with
- a first opening (2a) at a first end of the hollow body (1), and
- a second opening (2b) at a second end of the hollow body (1), and
- an adjustment means (4, 5) for adjusting the position of the device (10) in the body opening (ensuring fitting of the device (10) in the body opening, for adjusting penetration depth of the device in the body opening) positioned on an outer wall of the hollow body (1).

2. Device (10) according to claim 1, wherein the outer wall of the hollow body (1) is smooth for protecting the mucus membrane of the subject when in use.

3. Device (10) according to claim 1 or 2, wherein the first opening (2a) and the second opening (2b) are arranged such as to allow an air passage through the device (10).

4. Device (10) according to claims 1 to 3, wherein the hollow body (1) is preferably a hollow cylinder or a hollow truncated cone.

5. Device (10) according to claims 1 to 4, wherein the hollow body (1) tapers down at the first or second end to form a rounded tip (3).

6. Device (10) according to claims 1 to 5, wherein the adjustment means is a movable ring (4) positioned on the outer wall of the hollow body (1) such that it is movable on the outer wall of the hollow body (1) along a longitudinal axis of the hollow body (1).

7. Device (10) according to claim 6, wherein the movable ring (4) comprises a tongue (6b) for engaging into a groove (6a) positioned along a longitudinal axis of the hollow body (1) such that the tongue (6b) of the movable ring (4) moves along the groove (6a).

8. Device (10) according to claim 1 to 5, wherein the adjustment means is an adhesion means (5) on the outer wall of the hollow body (1).

9. Device (10) according to claim 1 to 8, comprising an absorbing material (7) for absorbing liquid, preferably a filter, inside the hollow body (1).

10. Device (10) according to claims 1 to 9, comprising a carrier substance (8) for binding liquids, preferably essential oils, inside the hollow body (1), preferably petroleum jelly, udder ointment, wax, or lanolin.

11. Device (10) according to claims 1 to 10, wherein a removable cap (9) is located at the first or second end of the hollow body (1) for preventing the escape of the chemical substance from the device.

12. Device (10) according to claims 1 to 11, preferably comprising or consisting of medical silicone for protecting the mucous membrane of the subject.

13. Process for producing a device (10) according to claims 1 to 12, comprising molding of
- a hollow body (1) for containing a volatile chemical substance (for inserting into a body opening of a subject), with
- a first opening (2a) at a first end of the hollow body (1), and
- a second opening (2b) at a second end of the hollow body (1), and of
- an adjustment means (4, 5) for adjusting the position of the device (1) in the body opening.

14. Use of the device (10) according to claims 1 to 12 for applying a volatile chemical substance into a body opening of a subject.
